# EUROPEAN PATENT SPECIFICATION

(11) **EP 3 943 949 B1**
(45) Date of publication and mention of the grant of the patent: **01.05.2024**
(21) Application number: 20783283.3
(22) Date of filing: 26.03.2020
(51) Int. Cl.: G01N 33/86, G01N 35/04

(54) **DETECTION DRIVE DEVICE AND DRIVE METHOD**
DETEKTIONSANTRIEBSVORRICHTUNG UND VERFAHREN
DISPOSITIF D'ENTRAÎNEMENT DE DÉTECTION ET PROCÉDÉ D'ENTRAÎNEMENT

(30) Priority: 29.03.2019 CN 201910251274
(43) Date of publication of application: 26.01.2022
(73) Proprietor: Hemoassay Science and Technology (Suzhou) Co., Ltd., Suzhou, Jiangsu 215000 (CN)
(72) Inventor: XIAO, Jian, Suzhou, Jiangsu 215000 (CN); HU, Xi, Suzhou, Jiangsu 215000 (CN); CHEN, Jienian, Suzhou, Jiangsu 215000 (CN); WANG, Tao, Suzhou, Jiangsu 215000 (CN); JIANG, Feng, Suzhou, Jiangsu 215000 (CN)
(74) Representative: Wang, Bo
(86) International application number: PCT/CN2020/081458
(87) International publication number: WO 2020/200045

(56) References cited:
- EP-A1- 0 976 448
- CN-A- 107 014 990
- US-A- 6 060 022
- US-A1- 2013 149 079
- US-A1- 2015 142 171
- US-B1- 6 293 750
- US-B2- 7 264 432

## Description

### TECHNICAL FIELD OF THE INVENTION

The invention relates to a driving device for a thromboelastometer.

### DESCRIPTION OF THE PRIOR ART

A thromboelastometer is a medical device used to test whether blood can coagulate normally. It is generally necessary to detect the patient's blood coagulation data through the thrombo elastometer before performing an operation, and judge whether the patient's blood coagulation process is normal based on the detected blood coagulation data. Only when the blood is able to coagulate normally, the patient can be operated on. If the blood coagulation process is abnormal, the patient's blood will not be able to coagulate normally during the operation, leading to difficulty in hemostasis, which may endanger the patient's life.

The physical properties of testing a blood clot are based on the following principles. A special cylindrical cup statically containing blood rotates at a low angular speed of 4.75, and a testing rod immersed in the blood sample is used to detect the movement of the blood sample. After the cup adheres to the testing rod through a fibrin platelet complex, the rotational force generated by rotating the cup can be transmitted to the testing rod in the blood sample. The strength of the fibrin platelet complex can influence the movement amplitude of the testing rod, so that a strong blood clot can synchronize the movements of both the testing rod and the cup. Therefore, the movement amplitude of the testing rod is directly related to the strength of the formed blood clot. In the case that the blood clot retracts or dissolves, the joint between the testing rod and the blood clot is released, so the movement of the cup is no longer transmitted to the testing rod, the rotation of which is detected by the sensor.

As for the current thromboelastometer, when detecting blood coagulation data, it is necessary to manually take cups, load cups, etc., which requires a lot of time for operators, resulting in high labor intensity and low test efficiency for detecting blood coagulation data. US6293750B1 discloses a driving device according to the preamble of claim 1.

### SUMMARY OF THE INVENTION

A objective of the invention is to overcome the defects of the prior art and provide a test-driving device and a driving method of the thromboelastometer that achieve automatically taking and loading cups.

To achieve the above object, the present invention provides a driving device according to claim 1.

Preferably, the up-down-driving mechanism includes a mounting frame, a second driving source, a driving steering assembly and a screw rod, the second driving source is fixedly installed on the mounting frame and connected with the driving steering assembly, the screw rod is installed on the mounting frame, connected with the driving steering assemble, and driven by the driving steering assemble to rotate, the screw rod is connected to the clamping-cup mechanism, while rotating, it drives the clamping-cup mechanism to move up and down in the first direction.

Preferably, the up-down-driving mechanism further includes at least one up-down-guiding rod fixed on the mounting frame, and the cup-clamping mechanism is connected to the up-down-guiding rod and moves up and down along the up-down-guiding rod.

Preferably, the cup-clamping mechanism further includes a sensor mounted on the clamping assembly-mounting seat and arranged close to the clamping jaw assembly, the sensor is used to test the state of clamping the testing cup.

Preferably, the rotationally-driving mechanism includes a third driving source and a rotating shaft connected to the third driving source, the rotating shaft is fixedly connected with the mounting plate of the up-down-driving mechanism to drive the cup-clamping mechanism to rotate in the second direction through the up-down-driving mechanism.

Preferably, the horizontally-driving mechanism includes a fourth driving source, a driving screw rod connected to the fourth driving source, and a slider connected to the driving screw rod, the slider is connected with the cup-clamping mechanism, so as to drive the cup-clamping mechanism to horizontally move in the second direction.

The beneficial effects of the invention are as follows. The invention achieves completely automatically taking and loading testing cups during detecting the blood coagulation data through the test-driving device (ie, mechanical hand), and automatically detecting the blood coagulation data by cooperating with the liquid-taking and test passage and other stations, thereby replacing manual operation, reducing the labor intensity of detecting blood coagulation data and the production cost. In addition, the mechanical hand runs reliably, and has good repeatability, and a low error rate, which improves test accuracy.

### BRIEF DESCRIPTION OF THE DRAWINGS

FIG 1 is a 3D schematic diagram of the invention (not including a rotationally-driving mechanism) at different angles.
FIG2 is a schematic diagram of the overall 3D mechanism of the invention except the horizontal drive mechanism.
FIG.3 is a top view of the structure of FIG 2.
FIG4 is a schematic diagram of the 3D structure of the cup-clamping mechanism and the up-down- driving mechanism of the invention after being assembled.
FIG.5 is a flow chart of the method according to the invention.

Wherein:
1- cup-clamping mechanism; 11- clamping assembly-mounting seat; 12- first driving source; 14- clamping jaw assembly; 141- clamping arm; 142- clamping arm; 15- resetting elastic member; 16- sensor; 2- up-down-driving mechanism; 21- mounting frame; 22- second driving source; 23- screw rod; 24- worm; 25- worm wheel; 26- up-down-guiding rod; 3-rotationally-driving mechanism; 31- third driving source; 32- rotating shaft; 4-horizontally-driving mechanism; 41- fourth driving source; 42- driving screw rod; 43- slider; 5-testing cup; 6- mounting plate.

### DETAILED DESCRIPTION OF THE INVENTION

The technical solutions of the embodiments in the invention will be clearly and completely described below in combination with the figures in the invention.

A test-driving device and a driving method disclosed in the invention achieve automatically taking, loading and unloading cups during detecting blood coagulation data by setting a cup clamping mechanism and a plurality of driving mechanisms matching with the mechanism.

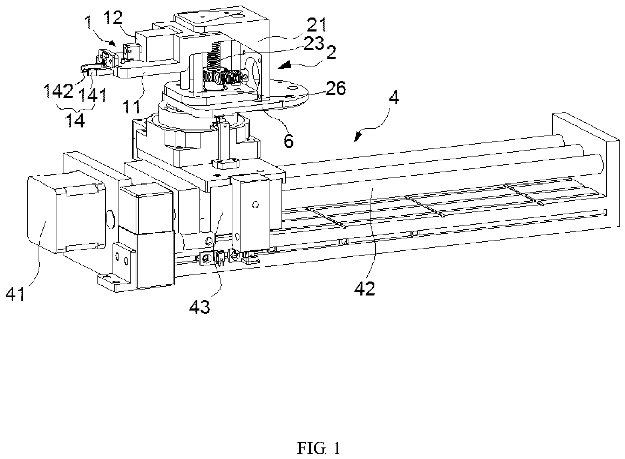

As shown in FIGs.1-2, the test-driving device disclosed in the embodiment according to the invention includes a cup-clamping mechanism 1, an up-down-driving mechanism 2, a rotationally-driving mechanism 3, and a horizontally-driving mechanism 4. The cup-clamping mechanism 1 is cooperatively driven by the up-down, rotationally and horizontally driving mechanisms 2, 3, 4 to clamp and unload the testing cup 5 to each station (not shown in the figures), which includes a cup tray, a test passage, and liquid-loading and waste-collecting stations, etc.

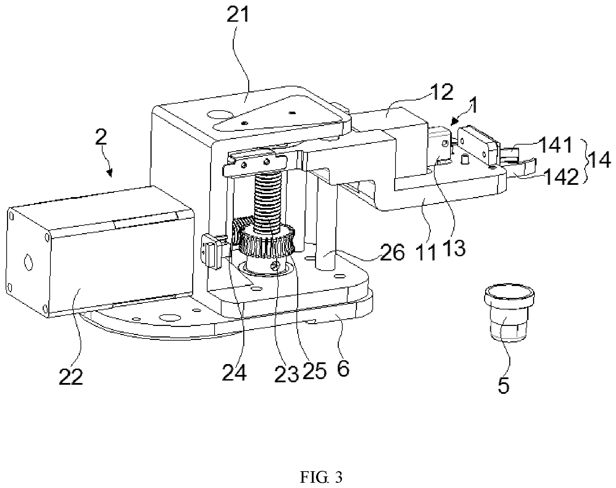

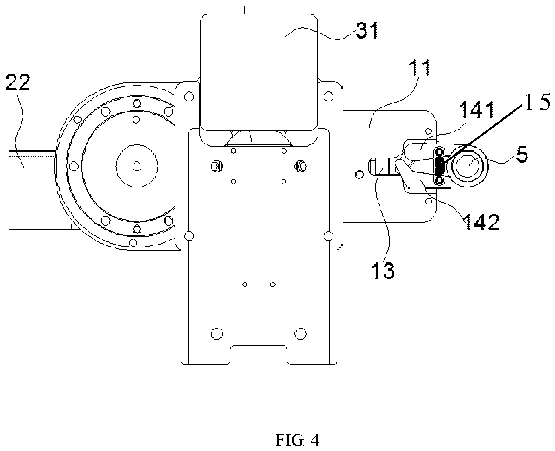

As shown in FIGs.3-4,the cup-clamping mechanism 1 is used to clamp and unload the testing cup 5, in this embodiment, it specifically includes a clamping assembly-mounting seat 11, a first driving source 12, a clamping jaw assembly 14 and a resetting elastic member 15, wherein the first driving source 12 is fixed on the clamping assembly-mounting seat 11, and moves back and forth in the horizontal direction, the clamping jaw assembly 14 is mounted on the clamping assembly-mounting seat 11, and clamps or unloads the testing cup 5 under the action of the first driving source 12, when implemented, the first driving source 12 can be an electromagnet. In this embodiment, the clamping jaw assembly 14 includes two clamping arms 141, 142, the ends of the two clamping arms 141, 142 cross with each other, and the other ends form a clamping jaw. The first driving source 12 moves in a direction close to the clamping jaw assembly 14 and drives the other end of the clamping jaw assembly 14 to open after abutting onto the end crossing with the clamping jaw assembly 14. The resetting elastic member 15 is arranged between the two clamping arms 141, 142 at the other end of the clamping jaw assembly 14, used to provide a resetting elastic force that causes the other ends of the two clamping arms 141, 142 to retract, when implemented, the resetting elastic member 15 may be a spring.

Preferably, the cup-clamping mechanism 1 further includes a sensor 16 mounted on the clamping assembly-mounting seat 11 and arranged close to the clamping jaw assembly 14. The sensor is used to test the state of clamping the testing cup 5 for the clamping jaw assembly 14, which has clamped the testing cup 5 or not.

As shown in FIGs.1,2,4, The up-down-driving mechanism 2 is connected to the cup-clamping mechanism 1 and is used to drive the cup-clamping mechanism 1 to move up and down in a first direction, in this embodiment, the first direction is the vertical direction. The up-down-driving mechanism 2 specifically includes a mounting frame 21, a second driving source 22, a driving steering assembly and a screw rod 23, wherein the second driving source 22 is fixedly installed on the mounting frame 21 and connected with the driving steering assembly, when implemented, the second driving source 22 can also be a motor, the driving steering assembly is used to convert the axial rotation of the driving shaft of the second driving source 22 into radial rotation, in this embodiment, the driving steering assembly includes a worm 24 connected to the driving shaft of the second driving source 22 and a worm wheel 25 matching and meshing with the worm 24, and the worm wheel 25 encircles the screw rod 23 and is threadedly connected with the screw 23, the screw rod 23 is vertically installed on the mounting frame 21 and driven to rotate in the horizontal direction by the worm wheel, the screw rod 23 is connected, for example, threadedly connected to the clamping assembly-mounting seat 11 of the clamping-cup mechanism 1, when the screw rod 23 rotates, the clamping assembly-mounting seat 11 is driven to move up and down in the vertical direction, thereby synchronously driving the clamping jaw assembly 14 to move up and down in the vertical direction. The testing cup 5 on the clamping jaw assembly 14 is adjusted to a desired height.

Preferably, the up-down-driving mechanism 2 further includes at least one up-down-guiding rod 26 vertically fixed on the mounting frame 21 for orientation, and the cup-clamping mechanism 1 is connected to the up-down-guiding rod 26. Specifically, the clamping assembly-mounting seat 11 of the clamping-cup mechanism 1 passes through the up-down-guiding rod 26, and is driven by the screw rod 23 to move up and down in the vertical direction along the up-down-guiding rod 26.

In addition, in this embodiment, the up-down-driving mechanism 2 and the rotationally-driving mechanism 3 are integrated and mounted on the same mounting plate 6, wherein the up-down-driving mechanism 2 is fixed above the mounting plate 6, and the rotationally-driving mechanism 3 is fixed below the mounting plate 6.

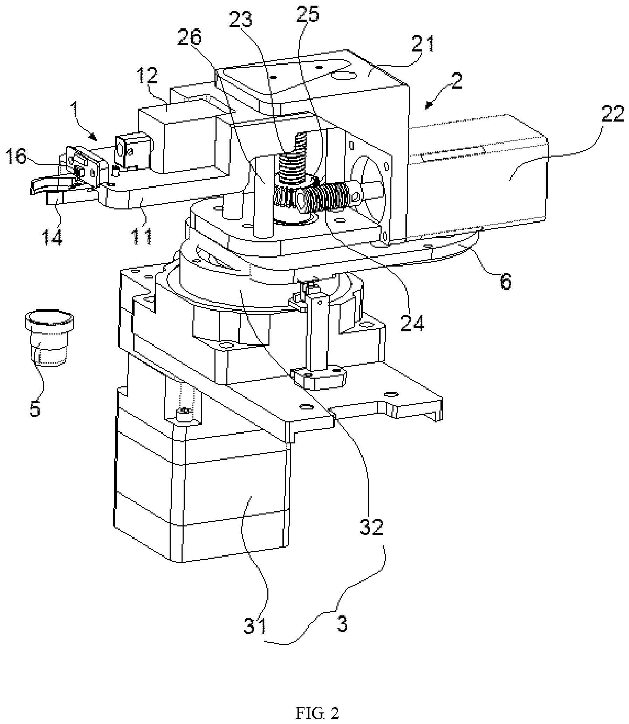

As shown in FIG2, the rotationally-driving mechanism 3 is connected to the cup-clamping mechanism 1, specifically connected to the mounting plate 6, used to drive the entire up-down-driving mechanism 2 to rotate in a second direction (the horizontal direction) perpendicular to the first direction through the mounting plate 6, further synchronously drive the cup-clamping mechanism 1 connected with the up-down-driving mechanism 2 to rotate in the horizontal direction, so that the cup-clamping mechanism 1 rotates to a desired position.

In this embodiment, the rotationally-driving mechanism 3 specifically includes a third driving source 31 and a rotating shaft 32 connected to the third driving source 31, when implemented, the third driving source 31 can also be a motor, the rotating shaft 32 is fixedly connected with the up-down-driving mechanism 2, specifically fixedly connected with the above-mentioned mounting plate 6, drives the up-down-driving mechanism 2 fixed on the mounting plate 6 by driving the mounting plate 6 to rotate in the horizontal direction, and then the up-down-driving mechanism 2 synchronously drives the cup-clamping mechanism 1 connected to it to rotate in the horizontal direction.

The horizontally-driving mechanism 4 is connected to the cup-clamping mechanism 1, specifically fixedly connected to the mounting plate 6, used to drive the cup-clamping mechanism 1 to move horizontally in the second direction perpendicular (the horizontal direction). Specifically, in this embodiment, the horizontally-driving mechanism 4 includes a fourth driving source 41, a driving screw rod 42 and a slider 43,wherein, when implemented, the fourth driving source 41 can also be a motor, the driving screw rod 42 is connected to the fourth driving source 41, the slider 43 is slidably mounted on the driving screw rod 42, and the upper end of the slider 43 is fixedly connected to the mounting plate 6, the driving screw rod 42 drives the slider 43 to move horizontally in the horizontal direction, driven by the fourth driving source 41, the slider 43 drives the rotationally-driving mechanism 3, the up-down-driving mechanism 2 and the cup-clamping mechanism 1 all integrated on the mounting plate 6 to slide in the horizontal direction, so as to make the cup-clamping mechanism 1 move to the corresponding station in the horizontal direction.

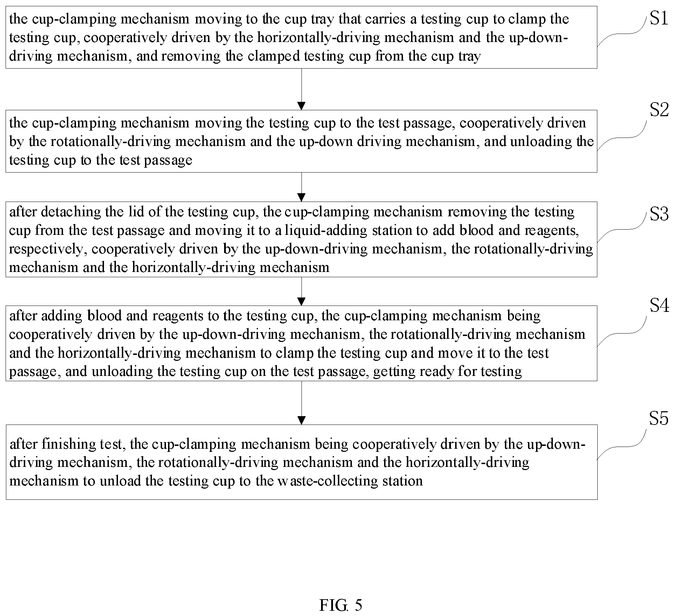

As shown in FIG 5, another technical solution disclosed in the embodiment according to the invention, test driving method, includes the following steps:
S1, The cup-clamping mechanism 1 moving to the cup tray (not shown in the figures) that carries the testing cup 5 to clamp the testing cup 5, cooperatively driven by the horizontally and up-down driving mechanisms 4, 2, and removing the clamped testing cup 5 from the cup tray.

Specifically, the cup-clamping mechanism 1 is driven by the horizontally-driving mechanism 4 to move to the top of the cup tray, so that the two clamping arms 141, 142 of the cup-clamping mechanism 1 are aligned with the testing cup 5 to be taken, after that, the first driving source 12 of the cup clamping mechanism 1 pushes the clamping jaw assembly 14 to open the two clamping arms 141,142 of the clamping jaw assembly 14, meanwhile, the up-down-driving mechanism 2 drives the clamping-cup mechanism 1 to descend, so that the cup wall of the testing cup 5 is located between both the clamping arms 141,142, after that, both the clamping arms 141,142 shrink and tighten the cup wall of the testing cup 5 under the elastic action of the resetting elastic member 15 to achieve clamping and picking the testing cup 5 on the cup tray.

After clamping the testing cup 5, the up-down-driving mechanism 2 drives the cup-clamping mechanism 1 to rise upwards, so that the clamped testing cup 5 is separated from the cup tray, which achieves removing the clamped testing cup 5 from the cup tray.

S2, The cup-clamping mechanism 1 moving the testing cup 5 to the test passage (not shown in the figures), cooperatively driven by the rotationally and up-down driving mechanisms 3,2, and unloading the testing cup 5 to the test passage.

Specifically, after the above step S1, the rotationally-driving mechanism 3 drives the cup-clamping mechanism 1 to rotate and move to the corresponding test passage from above the cup tray, so that the testing cup 5 is aligned with the cup-loading hole (not shown in the figures) of the corresponding testing cup 5 on the test passage, after that, the up-down-driving mechanism 2 drives the testing cup 5 to moves downwards and be loaded into the cup-loading hole. After that, the first driving source 12 drives the clamping-cup mechanism 1 to push the clamping jaw assembly 14 to open the two clamping arms 141, 142 of the clamping jaw assembly 1, so as to loosen the testing cup 5, and then the rotationally-driving mechanism 3 drives the clamping-cup mechanism 1 to rotate and move to other positions (the position that do not affect the operation of the test passage), and the test passage is expected to carry out the lid-up process.

S3, After detaching the lid of the testing cup 5, the cup-clamping mechanism 1 removing the testing cup 5 from the test passage and moving it to a liquid-adding station (not shown in the figures) to add blood and reagents, respectively, cooperatively driven by the up-down, rotationally and horizontally driving mechanisms 2, 3, 4.

Specifically, after the test passage completes carrying out the lid-up process, the lid of the testing cup 5 is detached from the testing cup 5, and the test passage conveys the testing cup 5 to the state to add liquid, then the cup-clamping mechanism 1 starts to work. First, the cup-clamping mechanism 1 rotates and moves to the test passage to align the clamping jaw assembly 14 with the testing cup 5, driven by the rotationally-driving mechanism 3. Next, the cup-clamping mechanism 1 pushes the clamping jaw assembly 14 to open the two clamping arms 141, 142 of the clamping jaw assembly 14, driven by the first driving source 12, meanwhile, the up-down-driving mechanism 2 drives the clamping-cup mechanism 1 to descend to make the cup wall of the testing cup 5 located between both the clamping arms 141,142. After that, both the clamping arms 141, 142 shrink and tighten the cup wall of the testing cup 5 under the elastic action of the resetting elastic member 15 to achieve clamping and taking the testing cup 5 on the test passage.

After clamping the testing cup 5, the up-down-driving mechanism 2 drives the cup-clamping mechanism 1 to rise upwards, so that the clamped testing cup 5 is separated from the cup-loading hole of the test passage, which achieves removing the clamped testing cup 5 from the test passage.

Afterwards, the cup-clamping mechanism 1 is cooperatively driven by the rotationally-driving mechanism 3 and the horizontally-driving mechanism 4 to transfer the testing cup 5 to the liquid-adding station for adding blood and reagents, respectively.

S4, After adding blood and reagents to the testing cup 5, the cup-clamping mechanism 1 being cooperatively driven by the rotationally, up-down and horizontally driving mechanisms 3, 2, 4 to clamp the testing cup 5 and move it to the test passage, and unloading the testing cup 5 on the test passage, getting ready for testing.

Specifically, after adding blood and reagents to the testing cup 5, the cup-clamping mechanism 1 is cooperatively driven by the rotationally-driving mechanism 3 and the horizontally-driving mechanism 4 to move the testing cup 5 to the test passage, so that the testing cup 5 is aligned with the cup-loading hole on the test passage, then the cup-clamping mechanism 1 moves downwards, driven by the up-down-driving mechanism 2, so that the testing cup 5 falls into the cup-loading hole, afterwards, the cup-clamping mechanism 1 pushes the clamping jaw assembly 14, driven by the first driving source 12, to open both the clamping arms 141, 142 of the clamping jaw assembly 14, both the clamping arms 141, 142 retract and reset under the elastic action of the resetting elastic member 15, meanwhile, the up-down-driving mechanism 2 drives the clamping arms 141, 142 of the cup-clamping mechanism 1, so as to first rise above the testing cup 5, and then descend, compressing the testing cup 5 to make it fixed in the cup-loading hole, and then the rotationally-driving mechanism 3 drives the clamping-cup mechanism 1 to rotate and move to other positions (the position that do not affect the operation of the test passage), and blood coagulation data is expected to be detected.

Furthermore, the driving method further includes:
S5, After finishing test, the cup-clamping mechanism 1 being cooperatively driven by the rotationally, up-down and horizontally driving mechanisms 3, 2, 4 to unload the testing cup 5 to the waste-collecting station (not shown in the figures).

How the rotationally, up-down and horizontally driving mechanisms 3, 2, 4 operate cooperatively and how the cup-clamping mechanism 1 operates to achieve unloading the testing cup to the waste-collecting station can refer to the description of S1~S4 above, and will not be repeated here. Further, it should be noted that as long as the cooperative process of the rotationally, up-down and horizontally driving mechanisms 3, 2, 4 in S1~S5 can achieve the process required by each step, the cooperative process is not limited to the action sequence described above.

The technical content and technical features of the invention have been disclosed as above, but a person skilled in the art may still make various replacements and modifications based on the teachings and disclosures of the invention without departing from the appended claims.

Therefore, the protection scope of the invention should not be limited to the content disclosed in the embodiments, but should include various replacements and modifications that do not deviate from the appended claims.

## Claims

1. A driving device for a thromboelastometer, wherein said device comprises a cup-clamping mechanism (1), and an up-down-driving mechanism (2), a rotationally-driving mechanism (3) and a horizontally-driving mechanism (4) that are connected to said cup-clamping mechanism (1),
wherein said up-down-driving mechanism (2) is used to drive said cup-clamping mechanism (1) to move up and down in a first direction,
said rotationally-driving mechanism (3) is used to drive said cup-clamping mechanism (1) to rotate in a second direction perpendicular to the first direction,
said horizontally-driving mechanism (4) is used to drive said cup-clamping mechanism (1) to move horizontally in the second direction,
said cup-clamping mechanism (1) is cooperatively driven by said rotationally-driving mechanism (3), said horizontally-driving mechanism (4) and said up-down-driving mechanism (2) to clamp and unload a testing cup (5) to each station of the thromboelastometer,
wherein said cup-clamping mechanism (1) includes a clamping assembly-mounting seat (11), a first driving source (12) fixed on said clamping assembly-mounting seat (11), a clamping jaw assembly (14) the driving device being **characterised in that** the said cup-clamping mechanism further comprises a resetting elastic member (15),
said clamping jaw assembly (14) includes two clamping arms (141, 142), the ends of the two clamping arms (141, 142) cross with each other, and the other ends form a clamping jaw, said driving device is configured such that the first driving source (12) moves in a direction close to the clamping jaw assembly (14) and drives the other end of the clamping jaw assembly (14) to open after abutting onto the end crossing with the clamping jaw assembly (14), said resetting elastic member (15) is connected with said clamping jaw assembly (14) to drive the other end of said clamping jaw assembly (14) to close and reset, and said first driving source (12) matches with the resetting elastic member (15) to drive said clamping jaw assembly (14) to clamp or loosen the testing cup (5).

2. The driving device according to claim 1, **characterized in that** said up-down-driving mechanism (2) includes a mounting frame (21), a second driving source (22), a driving steering assembly and a screw rod (23), said second driving source (22) is fixedly installed on said mounting frame (21) and connected with said driving steering assembly, said screw rod (23) is installed on said mounting frame (21), connected with said driving steering assemble, and driven by said driving steering assemble to rotate, said screw rod (23) is connected to said clamping-cup mechanism (1), while rotating, it drives said clamping-cup mechanism (1) to move up and down in the first direction.

3. The driving device according to claim 2, **characterized in that** said up-down-driving mechanism (2) further includes at least one up-down-guiding rod (26) fixed on the mounting frame (21), and said cup-clamping mechanism (1) is connected to said up-down-guiding rod (26) and moves up and down along said up-down-guiding rod (26).

4. The driving device according to claim 1, **characterized in that** said cup-clamping mechanism (1) further includes a sensor (16) mounted on said clamping assembly-mounting seat (11) and arranged close to said clamping jaw assembly (14), said sensor (16) is used to test the state of clamping the testing cup (5).

5. The driving device according to claim 2, **characterized in that** said rotationally-driving mechanism (3) includes a third driving source (31) and a rotating shaft (32) connected to said third driving source (31), said rotating shaft (32) is fixedly connected with the mounting plate (6) of said up-down-driving mechanism (2) to drive the cup-clamping mechanism (1) to rotate in the second direction through said up-down-driving mechanism (2).

6. The driving device according to claim 1, **characterized in that** said horizontally-driving mechanism (4) includes a fourth driving source (41), a driving screw rod (42) connected to said fourth driving source (41), and a slider (43) connected to said driving screw rod (42), said slider (43) is connected with said cup-clamping mechanism (1), so as to drive said cup-clamping mechanism (1) to horizontally move in the second direction.

## Patentansprüche

1. Antriebsvorrichtung für ein Thromboelastometer, wobei die Antriebsvorrichtung einen Becherklemmmechanismus (1) und einen Aufwärts/Abwärts-Antriebsmechanismus (2), einen Rotations-Antriebsmechanismus (3) und einen Horizontal-Antriebsmechanismus (4) umfasst, die mit dem Becherklemmmechanismus (1) verbunden sind,
wobei der Aufwärts/Abwärts-Antriebsmechanismus (2) verwendet wird, um den Becherklemmmechanismus (1) anzutreiben, damit er sich in einer ersten Richtung aufwärts und abwärts bewegt,
wobei der Rotations-Antriebsmechanismus (3) verwendet wird, um den Becherklemmmechanismus (1) anzutreiben, damit er sich in einer zweiten Richtung senkrecht zur ersten Richtung dreht,
wobei der Horizontal-Antriebsmechanismus (4) verwendet wird, um den Becherklemmmechanismus (1) anzutreiben, damit er sich horizontal in der zweiten Richtung bewegt,
wobei der Becherklemmmechanismus (1) durch den Rotations-Antriebsmechanismus (3), den Horizontal-Antriebsmechanismus (4) und den Aufwärts/Abwärts-Antriebsmechanismus zusammenwirkend angetrieben wird, um einen Testbecher (5) an jeder Station des Thromboelastometers zu klemmen und zu entladen,
wobei der Becherklemmmechanismus (1) einen Klemmbaugruppen-Montagesitz (11), eine erste Antriebsquelle (12), die an dem Klemmbaugruppen-Montagesitz (11) befestigt ist, und eine Klemmbackenbaugruppe (14) umfasst, **dadurch gekennzeichnet ist, dass** der Becherklemmmechanismus ferner ein elastisches Rückstellelement (15) umfasst, wobei die Klemmbackenbaugruppe (14) zwei Klemmarme (141, 142) umfasst, wobei die Enden der beiden Klemmarme (141, 142) einander kreuzen und die anderen Enden eine Klemmbacke bilden, wobei die Antriebsvorrichtung so konfiguriert ist, dass sich die erste Antriebsquelle (12) in eine Richtung nahe der Klemmbackenbaugruppe (14) bewegt und das andere Ende der Klemmbackenbaugruppe (14) antreibt, um sich zu öffnen, nachdem es an dem Ende anliegt, das sich mit der Klemmbackenbaugruppe (14) kreuzt, wobei das elastische Rückstellelement (15) mit der Klemmbackenbaugruppe (14) verbunden ist, um das andere Ende der Klemmbackenbaugruppe (14) zum Schließen und zum Rückstellen anzutreiben, und wobei die erste Antriebsquelle (12) mit dem elastischen Rückstellelement (15) zusammenwirkt, um die Klemmbackenbaugruppe (14) anzutreiben, um den Testbecher (5) zu klemmen oder zu lösen.

2. Antriebsvorrichtung nach Anspruch 1, **dadurch gekennzeichnet, dass** der Aufwärts/Abwärts-Antriebsmechanismus (2) einen Montagerahmen (21), eine zweite Antriebsquelle (22), eine Antriebslenkungsbaugruppe und eine Gewindestange (23) umfasst, wobei die zweite Antriebsquelle (22) fest an dem Montagerahmen (21) installiert und mit der Antriebslenkungsbaugruppe verbunden ist, wobei die Gewindestange (23) an dem Montagerahmen (21) installiert ist und mit der Antriebslenkungsbaugruppe verbunden ist und durch die Antriebslenkungsbaugruppe angetrieben wird, um sich zu drehen, wobei die Gewindestange (23) mit dem Becherklemmmechanismus (1) verbunden ist und bei Drehen den Becherklemmmechanismus (1) antreibt, um sich in der ersten Richtung aufwärts und abwärts bewegt.

3. Antriebsvorrichtung nach Anspruch 2, **dadurch gekennzeichnet, dass** der Aufwärts/Abwärts-Antriebsmechanismus (2) ferner mindestens eine Aufwärts/Abwärts-Führungsstange (26) umfasst, die an dem Montagerahmen (21) befestigt ist, und dass der Becherklemmmechanismus (1) mit der Aufwärts/Abwärts-Führungsstange (26) verbunden ist und sich entlang der Aufwärts/Abwärts-Führungsstange (26) aufwärts und abwärts bewegt.

4. Antriebsvorrichtung nach Anspruch 1, **dadurch gekennzeichnet, dass** der Becherklemmmechanismus (1) ferner einen Sensor (16) umfasst, der auf dem Klemmbaugruppen-Montagesitz (11) montiert und in der Nähe der Klemmbackenbaugruppe (14) angeordnet ist, wobei der Sensor (16) dazu dient, den Klemmzustand des Testbechers (5) zu prüfen.

5. Antriebsvorrichtung nach Anspruch 2, **dadurch gekennzeichnet, dass** der Rotations-Antriebsmechanismus (3) eine dritte Antriebsquelle (31) und eine mit der dritten Antriebsquelle (31) verbundene Drehwelle (32) umfasst, wobei die Drehwelle (32) fest mit der Montageplatte (6) des Aufwärts/Abwärts-Antriebsmechanismus (2) verbunden ist, um den Becherklemmmechanismus (1) so anzutreiben, dass er sich durch den Aufwärts/Abwärts-Antriebsmechanismus (2) in der zweiten Richtung dreht.

6. Antriebsvorrichtung nach Anspruch 1, **dadurch gekennzeichnet, dass** der Horizontal-Antriebsmechanismus (4) eine vierte Antriebsquelle (41), eine mit der vierten Antriebsquelle (41) verbundene Antriebsgewindestange (42) und einen mit der Antriebsgewindestange (42) verbundenen Schieber (43) umfasst, wobei der Schieber (43) mit dem Becherklemmmechanismus (1) verbunden ist, um den Becherklemmmechanismus (1) so anzutreiben, dass er sich in der zweiten Richtung horizontal bewegt.

## Revendications

1. Dispositif d'entraînement pour thromboélastomètre, dans lequel ledit dispositif comprend un mécanisme de serrage de coupelles (1), et un mécanisme d'entraînement de haut en bas (2), un mécanisme d'entraînement en rotation (3) et un mécanisme d'entraînement horizontal (4) qui sont reliés audit mécanisme de serrage de coupelles (1),
dans lequel ledit mécanisme d'entraînement de haut en bas (2) est utilisé pour entraîner ledit mécanisme de serrage de coupelles (1) à se déplacer vers le haut et vers le bas dans une première direction,
ledit mécanisme d'entraînement en rotation (3) est utilisé pour entraîner ledit mécanisme de serrage de coupelles (1) à tourner dans une seconde direction perpendiculaire à la première direction,
ledit mécanisme d'entraînement horizontal (4) est utilisé pour entraîner ledit mécanisme de serrage de coupelles (1) à se déplacer horizontalement dans la seconde direction,
ledit mécanisme de serrage de coupelles (1) est entraîné en coopération par ledit mécanisme d'entraînement en rotation (3), ledit mécanisme d'entraînement horizontal (4) et ledit mécanisme d'entraînement de haut en bas pour serrer et décharger une coupelle d'essai (5) à chaque station du thromboélastomètre,
dans lequel ledit mécanisme de serrage de coupelles (1) comprend un siège de montage de l'ensemble de serrage (11), une première source d'entraînement (12) fixée sur ledit siège de montage de l'ensemble de serrage (11), un ensemble de mâchoires de serrage (14), le dispositif d'entraînement étant **caractérisé en ce que** ledit mécanisme de serrage de coupelles comprend en outre un élément élastique de réarmement (15), ledit ensemble de mâchoires de serrage (14) comprend deux bras de serrage (141, 142), les extrémités des deux bras de serrage (141, 142) se croisent, et les autres extrémités forment une mâchoire de serrage, ledit dispositif d'entraînement est configuré de telle sorte que la première source d'entraînement (12) se déplace dans une direction proche de l'ensemble de mâchoires de serrage (14) et entraîne l'autre extrémité de l'ensemble de mâchoires de serrage (14) à s'ouvrir après avoir buté sur l'extrémité croisée avec l'ensemble de mâchoires de serrage (14), ledit élément élastique de réarmement (15) est relié à ladite mâchoire de serrage (14) pour entraîner l'autre extrémité de ladite mâchoire de serrage (14) à se fermer et à se réarmer, et ladite première source d'entraînement (12) correspond à l'élément élastique de réarmement (15) pour entraîner ladite mâchoire de serrage (14) à serrer ou à desserrer la coupelle d'essai (5).

2. Dispositif d'entraînement selon la revendication 1, **caractérisé en ce que** ledit mécanisme d'entraînement de haut en bas (2) comprend un cadre de montage (21), une deuxième source d'entraînement (22), un ensemble de direction d'entraînement et une tige filetée (23), ladite deuxième source d'entraînement (22) est installée de manière fixe sur ledit cadre de montage (21) et reliée à l'ensemble de direction d'entraînement, ladite tige de vis (23) est installée sur ledit cadre de montage (21), reliée audit ensemble de direction d'entraînement et entraînée en rotation par ledit ensemble de direction d'entraînement, ladite tige de vis (23) est reliée audit mécanisme de serrage de coupelles (1) et, en tournant, elle entraîne ledit mécanisme de serrage de coupelles (1) à se déplacer vers le haut et vers le bas dans la première direction.

3. Dispositif d'entraînement selon la revendication 2, **caractérisé en ce que** ledit mécanisme d'entraînement de haut en bas (2) comprend en outre au moins une tige de guidage de haut en bas (26) fixée sur le cadre de montage (21), et ledit mécanisme de serrage de coupelles (1) est relié à ladite tige de guidage de haut en bas (26) et se déplace de haut en bas le long de ladite tige de guidage de haut en bas (26).

4. Dispositif d'entraînement selon la revendication 1, **caractérisé en ce que** ledit mécanisme de serrage de coupelles (1) comprend en outre un capteur (16) monté sur ledit siège de montage de l'ensemble de serrage (11) et disposé à proximité dudit ensemble de mâchoires de serrage (14), ledit capteur (16) est utilisé pour tester l'état de serrage de la coupelle d'essai (5).

5. Dispositif d'entraînement selon la revendication 2, **caractérisé en ce que** ledit mécanisme d'entraînement en rotation (3) comprend une troisième source d'entraînement (31) et un arbre rotatif (32) relié à ladite troisième source d'entraînement (31), ledit arbre rotatif (32) est relié de manière fixe à la plaque de montage (6) dudit mécanisme d'entraînement de haut en bas (2) pour entraîner le mécanisme de serrage de coupelles (1) à tourner dans la deuxième direction par le biais dudit mécanisme d'entraînement de haut en bas (2).

6. Dispositif d'entraînement selon la revendication 1, **caractérisé en ce que** ledit mécanisme d'entraînement horizontal (4) comprend une quatrième source d'entraînement (41), une tige de vis d'entraînement (42) reliée à ladite quatrième source d'entraînement (41), et un curseur (43) relié à ladite tige de vis d'entraînement (42), ledit curseur (43) étant relié audit mécanisme de serrage de coupelles (1), de manière à entraîner ledit mécanisme de serrage de coupelles (1) à se déplacer horizontalement dans la seconde direction.
